# EUROPEAN PATENT APPLICATION

(11) **EP 2 253 275 A1**
(43) Date of publication of application: **24.11.2010**
(21) Application number: 10162414.6
(22) Date of filing: 10.05.2010
(51) Int. Cl.: A61B 8/14

(54) **Ultrasonic diagnostic apparatus, ultrasonic image processing apparatus and ultrasonic image processing method**

(30) Priority: 11.05.2009 JP 2009114815
(71) Applicant: Kabushiki Kaisha Toshiba, Tokyo 105-8001 (JP); Toshiba Medical Systems Corporation, Otawara-shi Toshigi 324-8550 (JP)
(72) Inventor: Okamura, Yoko, Otawara-shi Tochigi 324-8550 (JP); Kamiyama, Naohisa, Otawara-shi Tochigi 324-8550 (JP); Yoshida, Tetsuya, Otawara-shi Tochigi 324-8550 (JP)
(74) Representative: Kramer, Reinhold

(57) **Abstract**

According to one embodiment, an ultrasonic diagnostic apparatus (1) is disclosed. The ultrasonic diagnostic apparatus comprises an image data acquisition unit (21, 22, 23, 24) which transmits and receives an ultrasonic wave on a plurality of cross sections by selected one of ultrasonic scanning while swinging a one-dimensional array probe (12) and ultrasonic scanning using a two-dimensional array probe (12) thereby to acquire image data for each cross section, a reconstruction unit (25) which reconstructs volume data using a plurality of image data acquired by the ultrasonic scanning, an image generating unit (25) which generates at least one first image using the volume data and a plurality of second images corresponding to the cross section scanned by the ultrasonic wave using the plurality of image data acquired by the ultrasonic scanning, and a display unit (14) which displays the first image or the second image selectively, or displays the first image and the second image simultaneously.

## Description

Embodiments described herein relate generally to an ultrasonic diagnostic apparatus, an ultrasonic image processing apparatus and an ultrasonic image processing method.

The ultrasonic diagnosis is so convenient that the pulsation of a heart and the behavior of an embryo can be displayed in real time by a simple operation of applying an ultrasonic probe to the body surface on the one hand, and so high in safety that the inspection can be repeatedly conducted on the other hand. Also, the system size is small as compared with the other types of diagnostic apparatuses such as X-ray, CT and MRI, and therefore, the inspection can be easily conducted by bringing it to bedside. Further, unlike the X-ray diagnosis, the ultrasonic diagnosis is free of radiation exposure and can be used for obstetrics and home medication.

This ultrasonic diagnostic apparatus, like the X-ray mammography, is now widely used for diagnosis of the breast cancer. Also, the volume scan has been made possible by a mechanical probe with an ultrasonic transducer oscillated mechanically, with the result that a wide range of data can be obtained at a time, and a new image can be formed utilizing the data.

Typical images produced by the ultrasonic diagnostic apparatus are a B-mode tomogram and a three-dimensional reconstructed image. The B-mode image is obtained as an image mode used as a two-dimensional tomogram of the conventional ultrasonic diagnostic apparatus, and especially, can be visualized as a fine image of internal organs in a high-frequency band of 7 MHz to 10 MHz. Especially in recent years, the spatial resolution and the contrast resolution of this image have been improved further by such a technique as the harmonic imaging using the nonlinear phenomenon of sound wave. A three-dimensional reconstructed image, on the other hand, is formed using the image data on a plurality of cross sections obtained by the oscillatory scan (the ultrasonic wave is transmitted/received for a plurality of cross sections by ultrasonic scanning while swinging a one-dimensional array probe to obtain the image data for each cross section) thereby to generate the volume data. By using the volume data thus obtained, the three-dimensional reconstructed image is generated by the volume rendering process.
The use of this three-dimensional reconstructed image makes it possible to grasp the general shape of the internal organs three-dimensionally or to make satisfactory diagnosis in many situations including volume measurement.

Comparison between the image generated using the volume data (for example, the MPR image and the VR (volume rendering) image cut out as one cross section from the volume data, and hereinafter referred to as "the volume data-used image") and the B-mode tomogram obtained using the echo signal acquired by actual ultrasonic scanning of the same cross section (hereinafter referred to as "the scanning sectional image") shows that the spatial resolution and the contrast resolution of the volume data-used image is lower than those of the scanning sectional image. This is because the volume data-used image is generated through the image reconstruction process including the interpolation process.

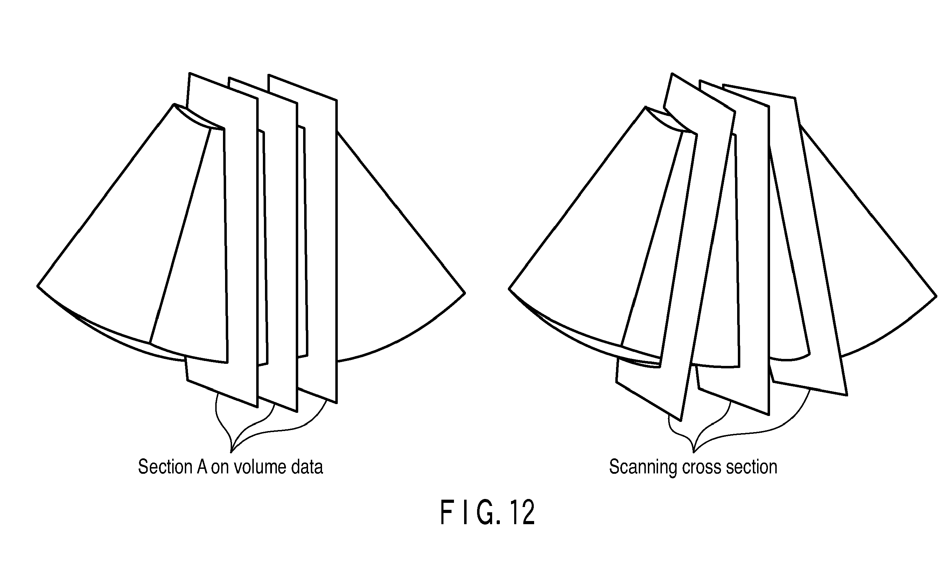

Specifically, in the case where the diagnosis of a fine structure is desired, for example, the scanning sectional image is much better in image quality, while the volume data-used image may not have a sufficient image equality. The conventional ultrasonic diagnostic apparatus, however, has no function of directly displaying (without executing the reconstruction process) the scanning sectional image acquired by the oscillatory scanning, nor can display, selectively or simultaneously, the volume data-used image and the scanning sectional image acquired by the oscillatory scanning. Further, the process such as the coordinate transform is executed in the reconstruction process.
As shown in FIG. 12, therefore, the position of a predetermined section before reconstruction (i.e. at the time of actual ultrasonic scanning) fails to correspond simply to that of the section A (surface A) of the same position on the volume data obtained by reconstruction. As a result, it is sometimes difficult for an operator to cut out the desired cross section (for example, the cross section containing a fine structure to be diagnosed) from the volume data with the sectional position for ultrasonic scanning as a clue.

The invention can be more fully understood from the following detailed description when taken in conjunction with the accompanying drawings, in which:
FIG. 1 is a diagram showing a block configuration of an ultrasonic diagnostic apparatus according to an embodiment;
FIG. 2 is a flowchart showing the flow of a process following display functions of a scanning sectional image and a volume data-used image in correspondence with each other;
FIG. 3 is a diagram showing an example of a VR image with a scanning sectional position containing information (position marker) indicating the position of the scanning sectional image in a volume rendering image;
FIG. 4 shows an example in which the scanning sectional image, the VR image with the scanning sectional position and a position indicator are displayed in parallel to each other on a monitor 14;
FIG. 5 shows an example in which the scanning sectional image, an MPR image (surfaces B and C) generated from the volume data, and the VR image with the scanning sectional position containing the scanning sectional position and a position marker indicating each position of the surfaces B and C are displayed in parallel to each other;
FIG. 6 shows an example in which the scanning sectional image, the MPR image for two cross sections orthogonal to the scanning sectional image, and the VR image with the scanning sectional position containing the scanning sectional position and the position marker indicating each position of the MPR images are displayed in parallel to each other;
FIG. 7 is a diagram showing a plurality of MPR images (image of surface A) displayed in multiple views;
FIG. 8 is a schematic diagram showing an area for displaying a scanning sectional image which is an area for displaying the scanning sectional image of the volume data and includes a selected scanning sectional image;
FIG. 9 shows an example in which the VR image with the scanning sectional position formed by using the extracted data corresponding to the area for displaying a scanning sectional image and the scanning sectional image included in the area for displaying a scanning sectional image are displayed in parallel to each other;
FIG. 10 is a diagram showing a C-mode image with thickness generated using the volume data;
FIG. 11 shows an example in which the VR image with the scanning sectional position formed by using the extracted data corresponding to the area for displaying a scanning sectional image and the scanning sectional image included in the area for displaying a scanning sectional image are displayed in parallel to each other; and
FIG. 12 is a diagram for explaining the relative positions of the scanning section before the reconstruction and the section (surface A) at the same position obtained on the volume data by the reconstruction.

In general, according to one embodiment, an ultrasonic diagnostic apparatus is disclosed. The ultrasonic diagnostic apparatus comprises: an image data acquisition unit which transmits and receives an ultrasonic wave on a plurality of cross sections by selected one of ultrasonic scanning while swinging a one-dimensional array probe or ultrasonic scanning using a two-dimensional array probe thereby to acquire image data for each cross section; a reconstruction unit which reconstructs volume data using a plurality of image data acquired by the ultrasonic scanning; an image generating unit which generates at least one first image using the volume data and a plurality of second images corresponding to the cross section scanned by the ultrasonic wave using said plurality of image data acquired by the ultrasonic scanning; and a display unit which displays the first image or the second image selectively, or displays the first image and the second image simultaneously.

Embodiments are described below with reference to the drawings. In the description that follows, the component elements having substantially the same function and configuration are designated by the same reference numeral and not explained again unless otherwise required. Also, to simplify the explanation in each embodiment, the object to be diagnosed is assumed to be the breast. In spite of this, the technical concept is also effectively applicable to predetermined internal organs such as the liver and pancreas as well as the breast.

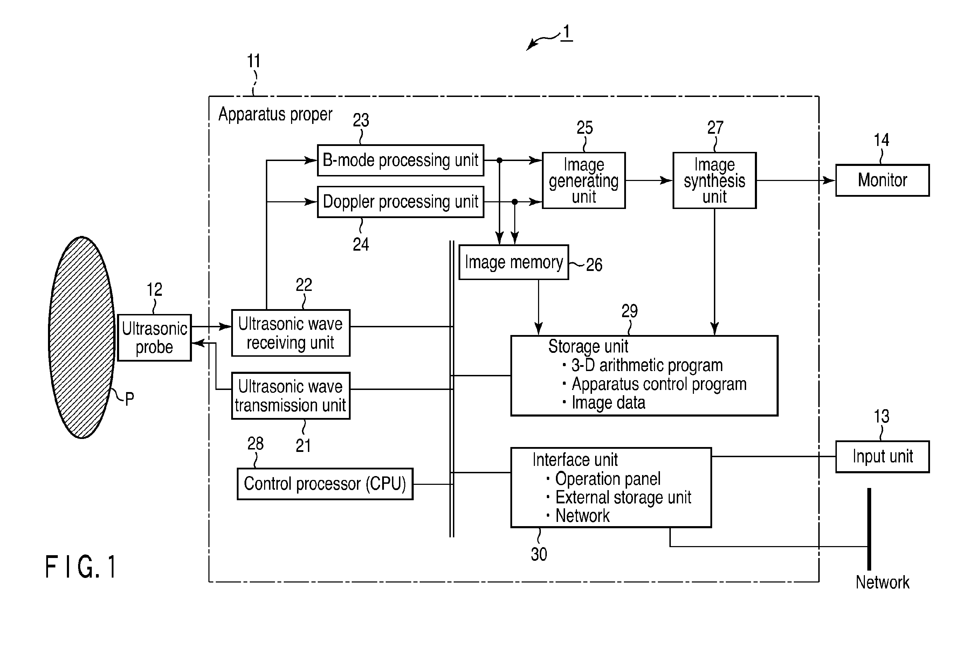

FIG. 1 is a diagram showing the block configuration of the ultrasonic diagnostic apparatus according to an embodiment. As shown in FIG. 1, an ultrasonic diagnostic apparatus 10 comprises an ultrasonic probe 12, an input unit 13 and a monitor 14, which are connected to an apparatus proper 11. The ultrasonic diagnostic apparatus 1 further comprises, as component elements built in the apparatus proper 11, an ultrasonic wave transmission unit 21, an ultrasonic wave receiving unit 22, a B-mode processing unit 23, a Doppler processing unit 24, an image generating unit 25, an image memory 26, an image synthesis unit 27, a control processor (CPU) 28, a storage unit 29 and an interface unit 30. The function of each component element is described below.

The ultrasonic probe 12 comprises a plurality of piezoelectric transducers for generating an ultrasonic wave based on a drive signal from the ultrasonic wave transmission unit 21 and converting the wave reflected from the subject into an electrical signal, a matching layer arranged in the piezoelectric transducers and a backing member for preventing the propagation of the ultrasonic wave from the piezoelectric transducers to the subsequent members. The ultrasonic wave, once transmitted from the ultrasonic probe 12 to a subject P, is reflected successively from the discontinuous surfaces having different acoustic impedances of the internal tissues and received by the ultrasonic probe 12 as an echo signal. The amplitude of the echo signal depends on the difference in acoustic impedance between the discontinuous surfaces reflecting the transmitted ultrasonic wave. Also, in the case where the transmitted ultrasonic pulse is reflected on the surfaces of the moving blood flow or the heart wall, the resulting echo is deviated in frequency in dependence on the velocity component of the mobile member in the direction of ultrasonic wave transmission due to the Doppler effect.

Incidentally, according to this embodiment, the ultrasonic probe 12, to be explained specifically, is assumed to be an oscillatory probe capable of ultrasonic scanning while mechanically oscillating a plurality of ultrasonic transducers along the direction perpendicular to a predetermined direction in which they are arranged.

The input unit 13 is connected to the apparatus proper 11 and comprises various switches, buttons, track balls, a mouse and a keyboard for retrieving various commands from the operator, instructions for setting the conditions and regions of interest (ROI) and various instructions to set the image quality conditions. Upon operation of an end button or a freeze button of the input unit 13 by the operator, for example, the transmission/reception of the ultrasonic wave is ended, and the operation of the ultrasonic diagnostic apparatus is suspended.

The monitor 14 displays the morphological information and the blood information in the living body as an image based on the video signal from the image generating unit 25.

The ultrasonic transmission unit 21 comprises a trigger generating circuit, a delay circuit and a pulsar circuit not shown. The pulsar circuit repeatedly generates the rate pulse to form the transmission ultrasonic wave at a predetermined rate frequency fr Hz (period: 1/fr seconds). In the delay circuit, the delay time required for condensing the ultrasonic wave into a beam for each channel and determining the transmission directivity is assigned to each rate pulse. The trigger generating circuit applies the drive pulse to the probe 12 at the timing based on the rate pulse.

Incidentally, the ultrasonic wave transmission unit 21 has the function of changing the transmission frequency and the transmission drive voltage instantaneously to execute a predetermined scan sequence in accordance with the command of the control processor 28. Especially, the transmission drive voltage can be changed by a transmission circuit of linear amplification type capable of switching the value of the transmission drive voltage instantaneously or a mechanism for electrically switching a plurality of power units.

The ultrasonic wave receiving unit 22 comprises an amplifier circuit, an A/D converter and an adder not shown. In the amplifier circuit, the echo signal retrieved through the probe 12 is amplified for each channel. In the A/D converter, the delay time required for determining the receiving directivity for the amplified echo signal is obtained, after which the add operation is performed on the adder. As a result of this add operation, the component reflected from the direction corresponding to the receiving directivity of the echo signal is highlighted, and a general beam for transmission and reception of the ultrasonic wave is formed by the receiving directivity and the transmission directivity.

The B-mode processing unit 23, by receiving the echo signal from the transmission unit 21, executes the process of logarithmic amplification and envelope detection thereby to generate signal intensity data in terms of brightness degree. In the image generating unit 25, the signal from the B-mode processing unit 23 is displayed on the monitor 14 as a B-mode image with the intensity of the reflected wave expressed as brightness. In the process, various image filtering operations such as edge highlighting, temporal smoothing and spatial smoothing are performed to provide the image quality meeting the user propensity.

The Doppler processing unit 24 analyzes the frequency of the speed information based on the echo signal received from the transmission unit 21, and by extracting an echo component of a contrast medium and the blood flow and the tissue due to the Doppler effect, determines the blood flow information such as average speed, dispersion and power at multiple points. The blood information thus obtained is sent to the image generating unit 25, and displayed in color on the monitor 14 as an average speed image, a dispersion image, a power image or any combination thereof.

In addition to these operations, the image generating unit 25 converts the scanning line signal train of ultrasonic scan into the scanning line signal train of an ordinary video format typically for TV, and generates the ultrasonic diagnostic image as a display image. The image generating unit 25 includes an exclusive processor and a memory for storing the image data, and by using these units, executes the coordinate transform process and the interpolation process to execute the reconstruction process for the three volume data. Further, the image generating unit 25 generates the scanning sectional image and the volume data-used image (MPR image, volume rendering image, etc.) in response to the command from the input unit 13. Incidentally, the data before entering the image generating unit 25 is sometimes called "raw data".

The image memory 26 is configured to store the ultrasonic wave image corresponding to a plurality of frames immediately before being frozen. By continuously displaying (cine display) the images stored in the image memory 26, the ultrasonic moving image can be displayed.

In the image synthesis unit 27, the image received from the image generating unit 25 is synthesized with text information of various parameters and scales, and output to the monitor 14 as a video signal. Also, the image synthesis unit 27 generates the VR image with the scanning sectional position containing the information indicating the position of the scanning sectional image in the volume rendering image.

The control processor 28 is a control unit having the function as an information processing device (computer) and controls the operation of the ultrasonic diagnostic apparatus proper. The control processor 28 reads, from the storage unit 29, a control program for image generation and display and an exclusive program for realizing the display function corresponding to the scanning sectional image and the volume data-used image. These programs are developed on the memory of the control processor 28 thereby to execute the arithmetic operation and the control operation for each process.

The storage unit 29 holds a control program for executing the transmission/receiving conditions, the image generation and the display process, diagnosis information (patient ID, doctor's opinion, etc.), a diagnosis protocol, a body mark generating program, an exclusive program for realizing the display function corresponding to the scanning sectional image and the volume data-used image described later, scanning sectional images corresponding to each frame, volume data and other data groups. Also, the storage unit 29 is used for holding the image in the image memory 26 as required. The data in the internal storage unit 29 can be transferred to an external peripheral device through the interface unit 30.

The interface unit 30 is associated with the input unit 13, the network and a new external storage unit (not shown). The data such as the ultrasonic image and the analysis result obtained by the apparatus can be transferred to other devices by the interface unit 30 through the network.

### (Display function corresponding to the scanning sectional image and the volume data-used image)

Next, the display function corresponding to the scanning sectional image and the volume data-used image of this ultrasonic diagnostic apparatus 1 is explained. According to this function, the scanning sectional image providing the B-mode image corresponding to the real scanning section(s) obtained by the ultrasonic scanning with the swinging of the one-dimensional array probe or by the ultrasonic scanning using the two-dimensional array probe is set in correspondence with the volume data-used image (volume rendering image, MPR image, etc.) generated by use of the volume data, and the resulting image is displayed at an arbitrary timing simultaneously or selectively. Incidentally, in the description that follows, an explanation is given specifically with reference to an example in which the one-dimensional array probe is used as the ultrasonic probe 12 and while swinging this one-dimensional array probe, the ultrasonic scanning is conducted to acquire the B-mode image corresponding to a plurality of cross sections.

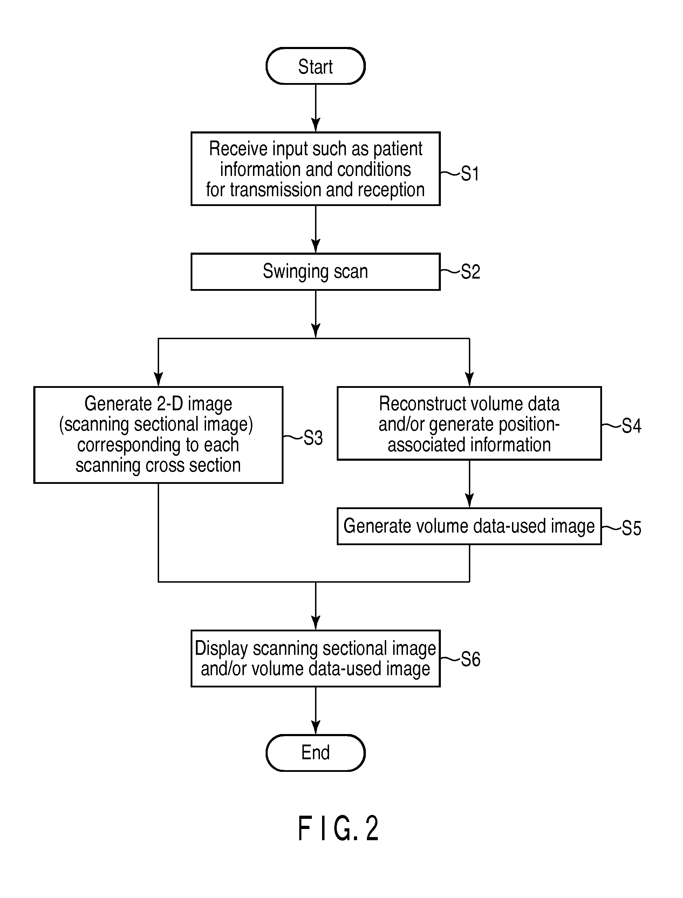

FIG. 2 is a flowchart showing the flow of the process following the display function corresponding to the scanning sectional image and the volume data-used image (the display process corresponding to the scanning section image and volume data-used image).
The process of each step is explained below.

### [Reception of input such as patient information and transmission/receiving condition, oscillatory scanning: steps S1, S2]

First, the patient information, the transmission/receiving conditions (focal depth, transmission voltage, oscillation range, etc.) are input through the input unit 13. Then, the control processor 28 stores the various information and conditions in the storage unit 29 (step S1). Next, the control processor 28 oscillates the ultrasonic transducer train of the ultrasonic probe 12 in the direction perpendicular to the direction of arrangement while at the same time transmitting the ultrasonic wave to each section corresponding to a plurality of oscillation angles (oscillating positions), and the ultrasonic scanning (oscillatory scanning) is repeatedly executed to acquire the echo signal from each section (step S2). As the result of this oscillatory scanning, the echo signal for a plurality of sections corresponding to each time point is obtained.

### [Generation of scanning sectional image: step S3]

The echo signal acquired for each section in step S2 is sent to the B-mode processing unit 23 through the ultrasonic wave receiving unit 22. The B-mode processing unit 23 executes the logarithmic amplification process, the envelope detection process, etc., and thus generates brightness data with the signal intensity expressed as brightness. The image generating unit 25 generates the two-dimensional image (scanning sectional image) corresponding to each scanned section using the brightness data received from the B-mode processing unit 23 (step S3).

### [Generation of volume data and information corresponding to position: step S4]

The image generating unit 25 reconstructs the volume data by executing the coordinate transform, with the interpolation process, from an actual spatial coordinate system (i.e. the coordinate system defining a plurality of scanning sectional image data) to a volume data spatial coordinate system for a plurality of scanning sectional image data generated. Also, the image generating unit 25 generates the information (information corresponding to the position, hereinafter referred to as "position-associated information") indicating the positional correspondence between the plurality of scanning sectional image data and the volume data (step S4). Incidentally, this position-associated information can be generated based on the relation between the states before and after the coordinate transform. The scanning sectional image data, the volume data and the position-associated information thus generated are stored in the storage unit 29.

### [Generation of volume data-used image: step S5]

The image generating unit 25 generates the volume data-used image such as the volume rendering image and the MPR image using the volume data generated (step S5).

### [Display of scanning sectional image, volume rendering image, etc.: step S6]

Next, with the volume rendering image on display, for example, assume that a command is issued through the input unit 13 to display the scanning sectional image. The control processor 28 performs the control operation to display the scanning sectional image and the volume rendering image.

Specifically, in the image synthesis unit 27, the VR image with the scanning sectional position containing the information indicating the position of the scanning sectional image in the volume rendering image is generated using the position-associated information and the volume rendering image under the control of the control processor 28. Further, the image synthesis unit 27 generates an indicator (position indicator) indicating the position of the scanned section (image) in the direction of oscillation direction as required. The control processor 28 displays according to a preset condition, on the monitor 14, the scanning sectional image, the volume data-used image, the correspondence image and the position color bar generated (step S6). The form in which these images are displayed is variously conceivable. Variations of the display form are explained below with reference to embodiments.

### (First example)

In the display form according to this example, an arbitrary volume rendering image providing a volume data-used image and an arbitrary scanning sectional image are displayed selectively (alternately, for example) at a desired timing according to an instruction input via the input unit.

The control processor 28 controls the image generating unit 25, the image synthesis unit 27 and the monitor 14 to display the arbitrary scanning sectional image and the arbitrary volume rendering image, each of which is selected by the user, at a desired timing. Each image can be displayed in a single-view form or a multi-view display form.

### (Second example)

The volume rendering image and the scanning sectional image are set in relative positions by the position-associated information. In the display form according to this example, when the volume rendering image providing a volume data-used image and the scanning sectional image are displayed selectively (alternately, for example), the scanning sectional image nearest to the volume rendering image is selected automatically.

For example, with the volume rendering image on display, a command is issued through the input unit 13 to display the scanning sectional image, and further, the desired position is designated through the input unit 13. The control processor 28 reads, from the storage unit 29, the scanning sectional image corresponding to the designated position in response to each command, and displays it by switching from the volume rendering image. In the absence of the scanning sectional image corresponding to the designated position, on the other hand, the control processor 28 generates the MPR image for the particular position using the volume data, and displays the MPR image generated by switching from the volume rendering image.

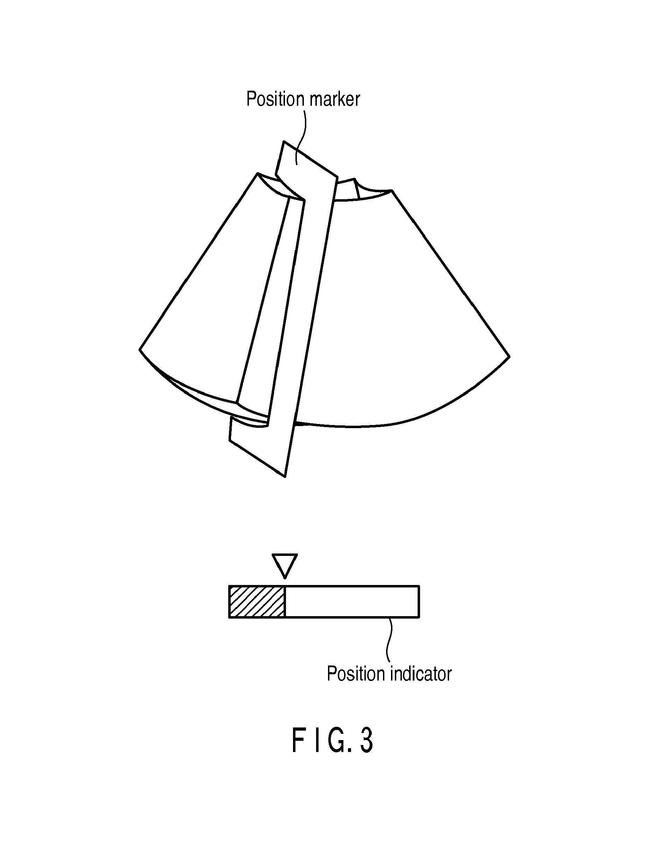

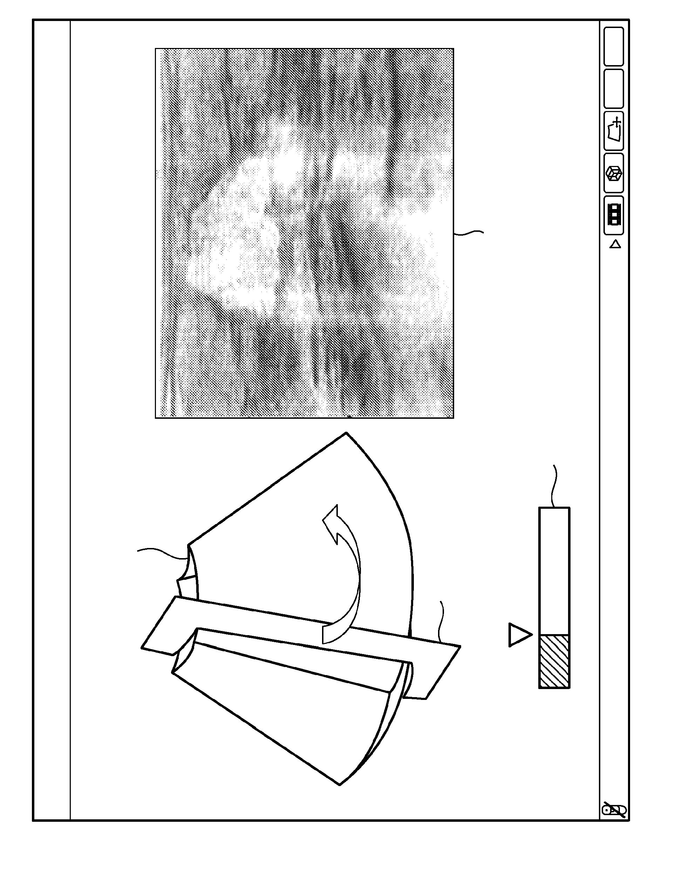

Incidentally, the VR image with the scanning sectional position containing the information (position marker) indicating the scanning sectional image in the volume rendering image may be displayed as a volume rendering image as shown in FIG. 3. Once the position of the position marker on the VR image with the scanning sectional position is designated, the scanning sectional image corresponding to the position marker is displayed, while upon designation of a position other than the position marker, the scanning sectional image corresponding to the designated position is displayed. At the same time, it is desirable to display the information for determining whether the ultrasonic image on display is the scanning sectional image or the MPR image generated from the volume data.

This image correspondence allows the operator to visually recognize, both accurately and quickly, the sectional position having the scanning sectional image and the sectional position not containing the scanning sectional image (i.e. the sectional position displayed as an MPR image).

Further, in the case where the VR image with the scanning sectional position is displayed, as shown in FIG. 3, the position indicator indicating the position of the scanning section in the direction of oscillation may be displayed. This position indicator permits the operator to visually recognize, both intuitively and quickly, the position of the scanning section in the direction of oscillation.

### (Third example)

The form of display according to this example represents a case in which the scanning sectional image and the VR image with the scanning sectional position providing the volume data-used image are displayed in parallel to each other.

FIG. 4 is a diagram showing an example of the form of display according to the third example, in which the scanning sectional image, the VR image with the scanning sectional position and the position indicator are displayed in parallel to each other on the monitor 14. The scanning sectional image and the VR image with the scanning sectional position may be displayed as a real-time moving image, a cine or a still image. Incidentally, this embodiment assumes a case in which the object to be diagnosed is the breast. In the case where the internal organ in periodic motion such as the heart is an object of diagnosis, however, the scanning sectional image and the VR image with the scanning sectional position are displayed in synchronism with each other.

### (Fourth example)

The form of display according to this example represents a case in which the scanning sectional image, two MPR images corresponding to two (for example, surfaces B and C) of the three orthogonal sections defined in the volume data and the VR image with the scanning sectional position are displayed in parallel to each other.

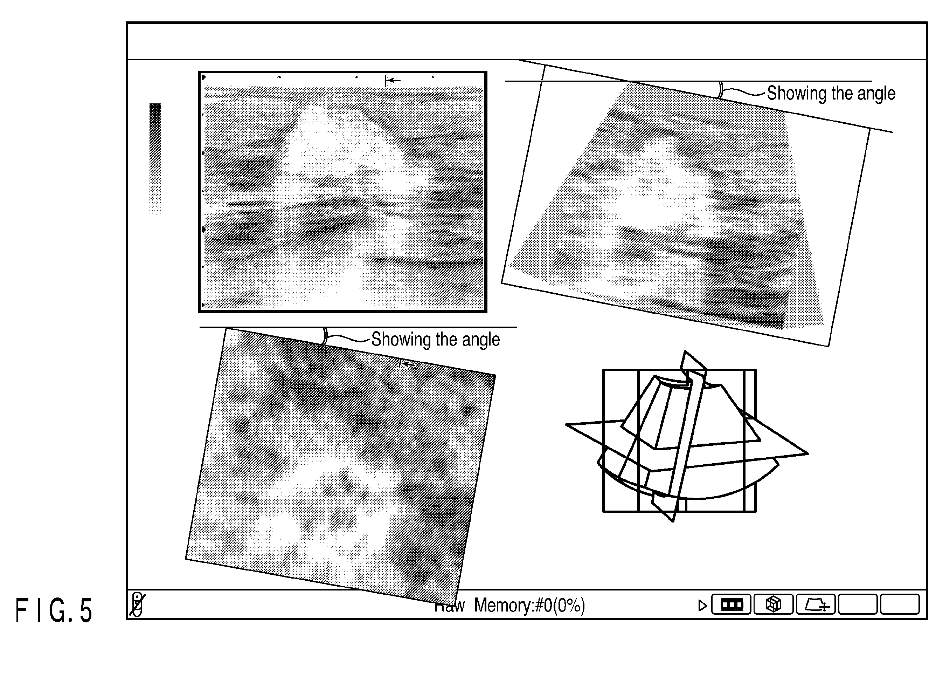

FIG. 5 is a diagram showing the form of display according to the fourth example, and represents a case in which the scanning sectional image, the MPR image (surfaces B and C) generated from the volume data and the VR image with the scanning sectional position containing the scanning sectional position and the position markers indicating the positions of the surfaces B and C are displayed in parallel to each other. According to this form of display, the scanning sectional image having a high spatial resolution and a high contrast resolution and the two MPR images corresponding to surfaces B and C can be observed at the same time while confirming the relative positions thereof.

Incidentally, as illustrated in FIG. 5, the MPR image corresponding to each of the surfaces B and C is displayed to indicate the position of the scanning sectional image and the position of the surface (hereinafter referred to as "the surface A'") perpendicular to the surface C, for example showing the angle between the scanning sectional image and the surface, and containing the line formed by the scanning sectional image crossing the upper surface (the surface nearest to the ultrasonic probe) of the volume. The operator can visually confirms, both quickly and easily, the degree of swinging of the oscillating operation from the relative positions of the scanning sectional image and the surface A' indicated in each MPR image corresponding to the surfaces B and C.

### (Fifth example)

The form of display according to this example represents a case in which the scanning sectional image, the two MPR images generated from the volume data and corresponding to the two cross sections orthogonal to the scanning sectional image and the VR image with the scanning sectional position are displayed in parallel to each other.

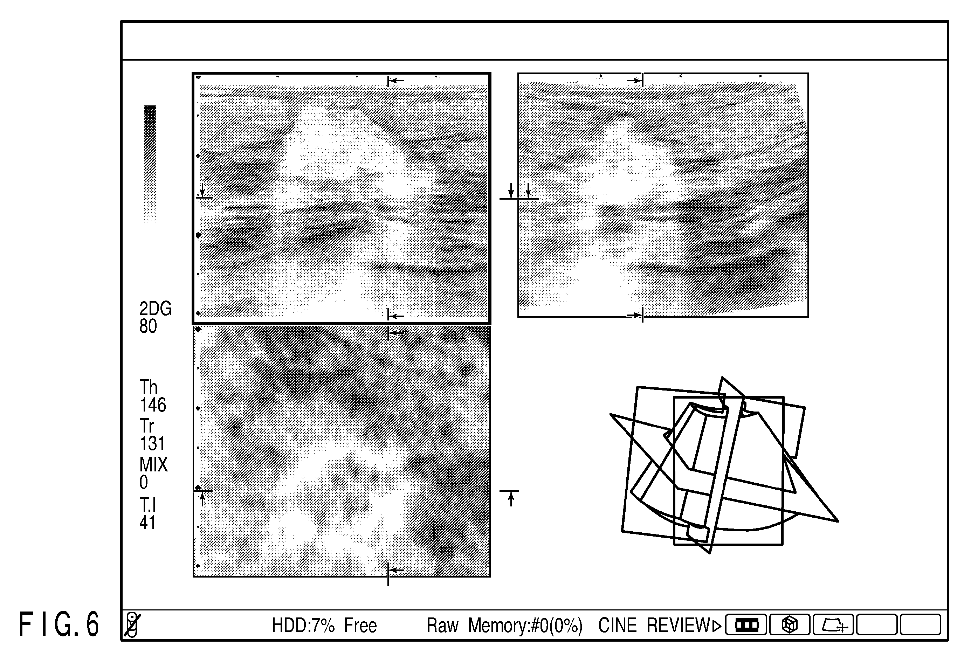

FIG. 6 is a diagram showing the form of display according to the fifth example, and represents a case in which the scanning sectional image, the MPR image for the two sections orthogonal to the particular scanning sectional image and the VR image with the scanning sectional position containing the scanning sectional position and the position marker indicating the position of each MPR image are displayed in parallel to each other. According to this form of display, the scanning sectional image having a high spatial resolution and contrast resolution and the two MPR images orthogonal to the scanning sectional image can be observed at the same time while confirming the relative positions thereof.

### (Sixth example)

The form of display according to this example represents a case in which a area of the volume data containing the scanning sectional image desirably studied in detail (area for displaying the scanning sectional image) utilizing the multi-view display (parallel display of multiple sections) of the MPR image or the scanning sectional image is extracted, and the scanning sectional image contained in the area for displaying the scanning sectional image and the VR image with the scanning sectional position corresponding to the extracted area for displaying the scanning sectional image are displayed in parallel to each other.

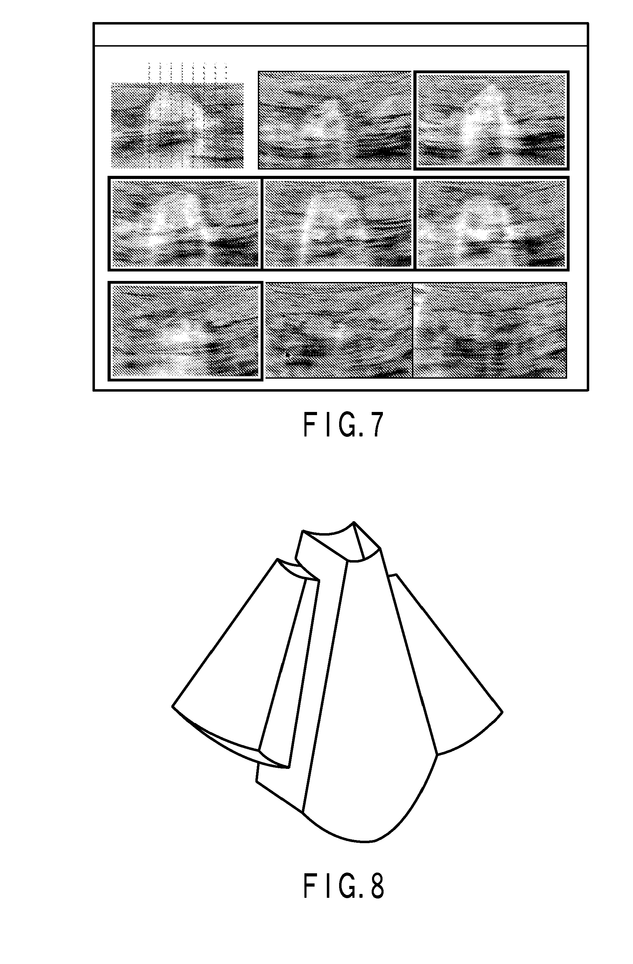

FIG. 7 is a diagram showing a plurality of MPR images (image of surface A) in multi-view display. The operator selects, through the input unit 13, the desired image as an object of detailed study from the scanning sectional images displayed in multiple views. This image selection can employ a method in which the two surfaces A corresponding to the two ends of the area for displaying the scanning sectional image, for example, or the image providing the center of the area for displaying the scanning sectional image is selected. The control processor 28, as shown in FIG. 8, extracts the area for displaying the scanning sectional image of the volume data containing the selected scanning sectional image, and using the data on the area for displaying the scanning sectional image thus extracted, generates the VR image with the scanning sectional position while at the same time displaying the scanning sectional image contained in the particular area for displaying the scanning sectional image.

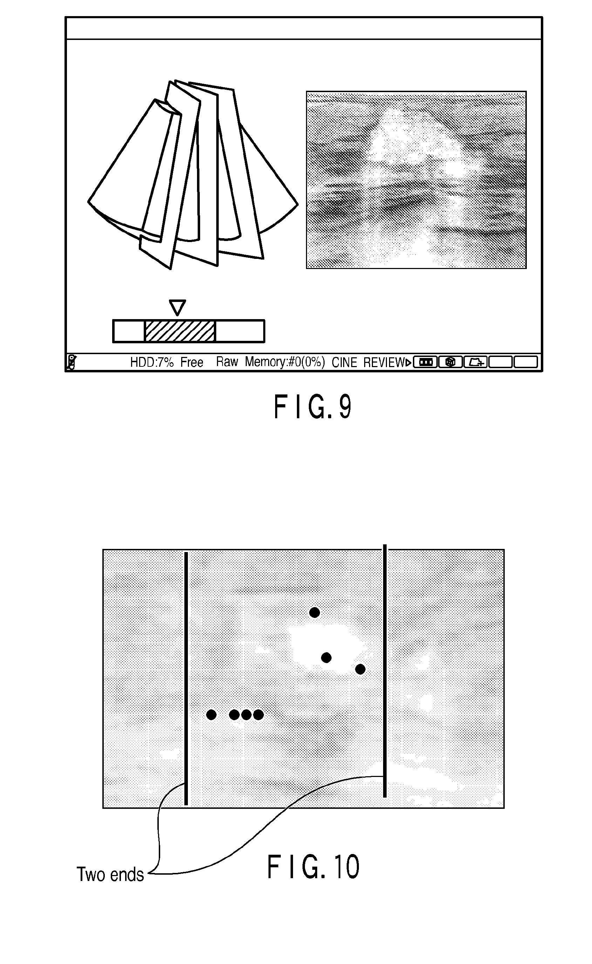

FIG. 9 is a diagram showing the form of display according to the fifth embodiment. As shown in FIG. 9, the VR image with the scanning sectional position and the scanning sectional image contained in the extracted area for displaying the scanning sectional image are displayed in parallel to each other using the data on the particular area for displaying the scanning sectional image. Also, the position indicator indicates the range of the extracted area for displaying the scanning sectional image. The operator can switch to and display the desired scanning sectional image by selecting, through the input unit 13, the position marker corresponding to the scanning sectional image contained in the VR image with the scanning sectional position.

### (Sixth embodiment)

According to the form of display according to this embodiment, the area for displaying the scanning sectional image of the volume data containing the scanning sectional image desirably studied in detail is extracted using the C-mode image with thickness, and the scanning sectional image contained in the area for displaying the scanning sectional image and the VR image with the scanning sectional position corresponding to the extracted area for displaying the scanning sectional image are displayed in parallel to each other.

FIG. 10 is a diagram showing the C-mode image with thickness generated using the volume data. The operator selects the width of extraction in the direction of oscillation of the area for displaying the scanning sectional image for the C-mode image with thickness through the input unit 13. This image is selected, as shown in FIG. 10, by selecting the two ends along the direction of oscillation of the area for displaying the scanning sectional image, for example, or the position constituting the center of the area for displaying the scanning sectional image. The control processor 28 extracts the area for displaying the scanning sectional image of the volume data containing the selected scanning sectional image as shown in FIG. 8, and generates the VR image with the scanning sectional position using the data on the extracted area for displaying the scanning sectional image while at the same time displaying the scanning sectional image contained in the particular area for displaying the scanning sectional image.

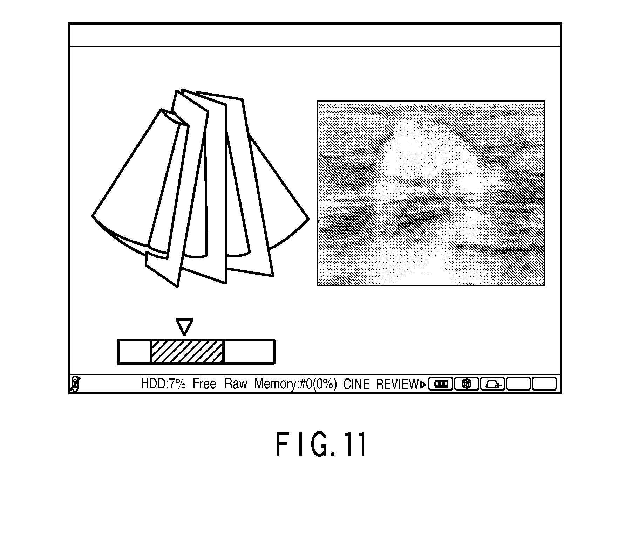

FIG. 11 is a diagram showing the form of display according to the sixth embodiment. As shown in FIG. 11, the VR image with the scanning sectional position and the scanning sectional image contained in the extracted area for displaying the scanning sectional image are displayed in parallel to each other using the data on the particular extracted area for displaying the scanning sectional image. Also, as in the fifth embodiment, the desired scanning sectional image can be switched to and displayed by selecting, through the input unit 13, the position marker corresponding to the scanning sectional image contained in the VR image with the scanning sectional position.

### (Effects)

The configuration described above can produce the effects described below.

With the ultrasonic diagnostic apparatus according to the embodiment, the scanning sectional image and the volume data-used image can be displayed selectively (alternately) at a desired timing according to an instruction input via the input unit. In addition, the scanning sectional image and the volume data-used image can be displayed simultaneously. Therefore, the general state of an internal organ can be easily grasped with the volume rendering image and the VR image with the scanning sectional position while at the same time observing the scanning sectional image for the desired position at the desired timing. As a result, with regard to the part requiring an especially detailed observation, the operator can selectively observe the scanning sectional image high in spatial resolution and contrast resolution, thereby making it possible to realize the image diagnosis of higher quality.

Also, in the ultrasonic diagnostic apparatus according to this embodiment, the area to be diagnosed in detail using the volume data-used image can be designated and the scanning sectional image contained in the designated area can be selectively displayed. The observer, therefore, can quickly and accurately select a diagnosis area and the scanning sectional image corresponding to the particular diagnosis area. As a result, the work load on the observer for image diagnosis is reduced.

Incidentally, the embodiments are not limited to the embodiments described above, but can be embodied by modifying the component elements thereof without departing from the spirit and scope of the embodiments. Specific examples of modifications are described below.
(1) Each function of the embodiments can be realized also by installing an execution program for the processes in a computer such as a work station and developing it on a memory. At the same time, the program adapted to cause the computer to carry out the method can be distributed by being stored in a recording medium such as a magnetic disk (Floppy (registered trademark) disk, hard disk, etc.), optical disk (CD-ROM, DVD, etc.) or a semiconductor memory.
(2) Although the embodiments described above represent a case in which the display function corresponding to the scanning sectional image and the volume data-used image is realized by the ultrasonic diagnostic apparatus, the embodiments is not limited to such a case, and the display function corresponding to the scanning sectional image and the volume data-used image can be realized ex post facto with the ultrasonic image processing apparatus, for example, by storing the data, the volume data and the position-associated information for the scanning sectional image in a storage unit.
(3) Although the embodiments described above represent a case in which, as shown in FIG. 2, the scanning sectional image, the volume data and the volume data-used image are generated in parallel to each other, the timing of generating the scanning sectional image is not limited to the aforementioned case. Instead, the raw data of the scanning sectional image may be stored as it is in the storage unit 29, for example, and in response to the designation of the section of the volume data-used image such as the VR image with the scanning sectional position, the raw data corresponding to the section involved may be read each time thereby to generate and display the scanning sectional image.
(4) Although the embodiments described above represent a case in which the ultrasonic probe is an oscillatory probe, the ultrasonic probe 12 is not limited to the oscillatory probe, but any probe may be employed which can collect a plurality of two-dimensional image data, such as the two-dimensional array probe (the probe with ultrasonic transducers two-dimensionally arranged in matrix), the multiplane probe or the one-dimensional array probe capable of ultrasonic scan while being manually shaken. In any of these probes, the display function corresponding to the scanning sectional image and the volume data-used image described above can be realized by positional correspondence between the scanning sectional image and the volume data.

Also, various modifications of the embodiments can be realized by appropriately combining a plurality of the component elements disclosed in the embodiments above. For example, some of the component elements included in the embodiments may be deleted. Further, the component elements of different embodiments may be combined with each other appropriately.

As described above, according to this embodiment, an ultrasonic diagnostic apparatus, an ultrasonic image processing apparatus and an ultrasonic image processing method are realized in which the B-mode image high in spatial resolution and contrast resolution and the image generated using the volume data can be discriminated from each other, displayed selectively (alternately) at a desired timing and displayed in a predetermined form at the desired timing selectively or in correspondence with each other.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

## Claims

1. An ultrasonic image processing apparatus (1) comprising:
a storage unit (29), which is configured to store image data acquired for each cross section by transmitting and receiving an ultrasonic wave on a plurality of cross sections by selected one of ultrasonic scanning while swinging a one-dimensional array probe or ultrasonic scanning using a two-dimensional array probe;
a reconstruction unit (25), which is configured to reconstruct volume data using a plurality of image data acquired by the ultrasonic scanning;
an image generating unit (25), which is configured to generate at least one first image using the volume data and a plurality of second images corresponding to the cross sections scanned by the ultrasonic wave using said plurality of image data acquired by the ultrasonic scanning; and
a display unit (14), which is configured to display the first image or the second image selectively, or display the first image and the second image simultaneously.

2. The ultrasonic image processing apparatus according to claim 1, further comprising a input unit (13), which is configured to input an instruction of selecting the first image or the second image when the first image or the second image is displayed selectively.

3. The ultrasonic image processing apparatus according to claim 1 or 2, further comprising:
a positional correspondence processing unit, which is configured to set the volume data in positional correspondence with said plurality of image data acquired by the ultrasonic scanning;
wherein the at least one first image includes a volume rendering image, and
the display unit (14) is configured to display the second image corresponding to a position designated for the volume rendering image.

4. The ultrasonic image processing apparatus according to any one of claims 1 to 3, **characterized in that** said at least one first image includes an image corresponding to the cross section orthogonal to the second image displayed.

5. The ultrasonic image processing apparatus according to any one of claims 1 to 4, **characterized in that** said at least one first image includes an image corresponding to two of three orthogonal cross sections defined in the volume data.

6. The ultrasonic image processing apparatus according to claim 5, **characterized in that** the display unit (14) is configured to display the position of the second image displayed and the position of the remaining one of the three orthogonal cross sections on the image corresponding to the two of the three orthogonal cross sections.

7. The ultrasonic image processing apparatus according to any one of claims 1 to 6, further comprising a designation unit (25, 28), which is configured to designate a predetermined area for the volume data,
wherein the image generating unit (25) is configured to generate the second image included in the predetermined area designated by the designation unit.

8. An ultrasonic diagnostic apparatus (1) comprising:
an image data acquisition unit (21, 22, 23, 24) which is configured to transmit and receive an ultrasonic wave on a plurality of cross sections by selected one of ultrasonic scanning while swinging a one-dimensional array probe (12) or ultrasonic scanning using a two-dimensional array probe (12) thereby to acquire image data for each cross section; and
an ultrasonic image processing apparatus according to any one of claims 1 to 7.

9. An ultrasonic image processing method comprising:
transmitting and receiving an ultrasonic wave on a plurality of cross sections by selected one of ultrasonic scanning while swinging a one-dimensional array probe or ultrasonic scanning using a two-dimensional array probe thereby to acquire image data for each cross section;
reconstructing volume data using a plurality of image data acquired by the ultrasonic scanning;
generating at least one first image using the volume data and a plurality of second images corresponding to the cross section scanned by the ultrasonic wave using said plurality of image data acquired by the ultrasonic scanning; and
displaying the first image or the second image selectively, or displaying the first image and the second image simultaneously.

10. The ultrasonic image processing method according to claim 9, further comprising inputting an instruction of selecting the first image or the second image when the first image or the second image is displayed selectively.

11. The ultrasonic image processing method according to claim 9 or 10, further comprising:
setting the volume data in positional correspondence with said plurality of image data acquired by the ultrasonic scanning;
wherein said at least one first image includes a volume rendering image, and
the second image corresponding to a position designated for the volume rendering image is displayed.

12. The ultrasonic image processing method according to any one of claims 9 to 11, **characterized in that** said at least one first image includes an image corresponding to the cross section orthogonal to the second image displayed.

13. The ultrasonic image processing method according to any one of claims 9 to 12, **characterized in that** said at least one first image includes an image corresponding to two of three orthogonal cross sections defined in the volume data.

14. The ultrasonic image processing method according to claim 13, further comprising: displaying the position of the second image displayed and the position of the remaining one of the three orthogonal cross sections on the image corresponding to the two of the three orthogonal cross sections.

15. The ultrasonic image processing method according to any one of claims 9 to 14,
**characterized in that** the second image included in a predetermined area designated for the volume data is generated in the image generating step.
